(19) **Europäisches Patentamt**
**European Patent Office**
**Office européen des brevets**

(11) **EP 2 021 765 B1**

(12) **EUROPEAN PATENT SPECIFICATION**

(45) Date of publication and mention
of the grant of the patent:
**21.07.2010 Bulletin 2010/29**

(51) Int Cl.:
***G01N 15/08*** (2006.01)

(21) Application number: **07732697.3**

(86) International application number:
**PCT/GB2007/001668**

(22) Date of filing: **04.05.2007**

(87) International publication number:
**WO 2007/129076 (15.11.2007 Gazette 2007/46)**

(54) **IMAGING SYSTEM FOR DETERMINING PERMEABILITY OF PAPER**

BILDERZEUGUNGSSYSTEM ZUR BESTIMMUNG DER DURCHLÄSSIGKEIT VON PAPIER

SYSTÈME D'IMAGERIE DESTINÉ À DÉTERMINER LA PERMÉABILITÉ DU PAPIER

(84) Designated Contracting States:
**AT DE FR GB IT**

(30) Priority: **09.05.2006 GB 0609184**

(43) Date of publication of application:
**11.02.2009 Bulletin 2009/07**

(73) Proprietor: **Molins PLC**
**Linford Wood East,**
**Milton Keynes,**
**Bucks, MK14 6LY (GB)**

(72) Inventors:
• **TINDALL, Ian Francis**
**Bournemouth**
**Dorset BH9 2UJ (GB)**

• **TWELFTREE, Martin Oliver**
**Springfield**
**Milton Keynes MK6 3LD. (GB)**

(74) Representative: **Williams, Michael Ian et al**
**Cleveland**
**40-43 Chancery Lane**
**London WC2A 1JQ (GB)**

(56) References cited:
**EP-A- 1 783 080     US-A- 4 628 468**
**US-A- 5 341 824     US-B1- 6 415 045**

Printed by Jouve, 75001 PARIS (FR)

**Description**

This invention relates to techniques for determining the permeability of a sample of paper, such as paper used in the manufacture of cigarettes.

There are many different types of papers used in the manufacture of cigarettes, and besides features associated with aesthetics, each paper has a particular function within the design. In particular, these papers are used to control the burning characteristics of the cigarette and the dilution of smoke delivered to the user.

Cigarette papers typically allow some air to pass through the paper and into the cigarette as air is drawn through the rod. Papers can also be deliberately perforated to allow more air to pass into the cigarette and thereby to change the delivery of tar and nicotine. A measure of the porosity of paper is given by paper permeability; this is usually expressed as a flow rate per unit area given a standard pressure difference across the paper boundary. In the situation where the paper contains holes, the total permeability will be contributed to by the intrinsic permeability of the paper, and any holes present. Permeability will be high where the total area of holes is large.

Cigarette tipping paper surrounds, and extends beyond, the filter and often includes deliberate holes. These holes dilute the delivery of smoke to the user and affect the "taste" of the cigarette. It is desirable that the permeability of tipping papers is monitored closely since if papers do not have the expected porosity then the delivery of smoke will not be as desired and the cigarette "taste" may not be as expected.

Cigarette envelope paper surrounds the tobacco, and any unusual porosity in this paper will change the dilution of the smoke and also change the burning characteristics of the lit coal. In turn, this is likely to change the chemical composition of the trace smoke constituents; these form as a result of pyrolysis and the temperature of pyrolysis can affect their formation.

For these reasons it can be seen that it is desirable for the papers used in the manufacture of cigarettes to have a known and controlled permeability. This is particularly important from a safety and regulatory point of view since inconsistencies in the paper permeability could give rise to an undesirable delivery of tar and nicotine to the user.

Known techniques for measuring the permeability of paper, and in particular cigarette papers, primarily revolve around the use of differential pressure systems. Such systems are expensive and difficult to maintain. For these reasons it is impossible, for reasons of practicality, to use pressure differential systems to measure the permeability of all paper used in the manufacture of cigarettes. Therefore, only samples are ever analysed, which can lead to faulty papers being used in cigarette products.

US patent no. US5,341,824 describes a system for inspecting and controlling the production of perforated paper, using optical measurements correlated to pressure drop.

It is an object of the present invention to provide a system and method for quickly and inexpensively determining the permeability of a sample of paper.

According to a first aspect of the present invention there is provided a permeability determination system as specified in claim 1.

The means for determining the brightness of the image and the data analysis means for determining the permeability of the sample of paper may be, for example, a processor, or control electronics; any routines may be implemented in software or in hardware. Such a processor could be integrated in the imaging device or, in just one alternative, could be located in an external analysis unit such as a personal computer.

The invention may provide the advantage that the permeability of a sample of paper may be measured quickly, based only on the brightness of an image of that sample. This allows a measurement of permeability which is comparatively inexpensive, as oppose to alternative permeability measuring techniques involving the use of pressure differential equipment. In addition, any average camera would suffice since only the brightness of the image need be determined. Therefore the camera need not have high resolution or even be in focus.

Such a measurement of permeability may be of particular value when measuring the permeability of cigarette paper since any inconsistency in permeability could give rise to an inconsistent delivery of tar and nicotine to the user. The paper used may have an intrinsic, non-zero, permeability which contributes to the overall permeability of the sample of paper; such papers are common in cigarette filters.

The means for determining the brightness of the image further determine differences in brightness in the image, and the means for determining the permeability of the sample of paper does so based on the differences in brightness.

Areas in the image that contrast with the normal background brightness of the sample of paper correspond to perforations in the paper. In addition the size and relative brightness of these areas may be related to the area of hole imaged. Thus by analysing areas of different brightness, the permeability of the sample of paper may be determined. This allows a more accurate determination of the permeability of the sample of paper that remains fast and inexpensive.

There are data analysis means for detecting at least one area in the image which corresponds to a hole in the sample of paper; and also the data analysis means for determining the permeability of the sample of paper is arranged to determine the contribution of detected holes to the permeability of the sample of paper.

By detecting areas in the image that correspond to holes, specific areas in the image can be linked to actual holes in the sample of paper. Any holes present in the paper act to increase the permeability of the sample in a deterministic way. Thus the contribution of any holes to the permeability of the paper may be determined in order to find the permeability of the sample. This is particularly appropriate in the measurement of cigarette papers, some of which may be deliberately perforated.

Preferably the permeability determination system further comprises data analysis means for determining the area of detected holes. This can allow the permeability to be determined based on the size of the holes as well as the number of holes, which may provide a more accurate determination of the permeability. In addition, or alternatively, there may be data analysis means for determining the shape of detected holes. This may be important as holes that have the same area may have different effects on the permeability of a sample of paper. For example, slits in the paper may have a low area but a comparatively large effect in increasing the permeability of the sample of paper.

The magnification factor for images produced by the imaging device may be variable. This can allow the imaging device to "zoom in" on areas of interest, and particularly holes, in the image. Once identified and located in a first image, the imagining device may increase the magnification factor for images produced in order to "zoom in" on a hole. This will increase the resolution of the paper sample in the image and may be particularly useful for determining the shape and area of detected holes. Alternatively, rather than using a zoom system, the magnification factor may be varied by simply changing the lens in the imaging device.

The permeability determination system may further comprise means for projecting radiation on the sample of paper (such as a radiation emitter or a light). By projecting radiation onto the paper any holes that are present will be highlighted, and accordingly it will be easier to detect areas in the image which correspond to holes. The radiation projected on the sample of paper may be visible light. By using visible light, a conventional optical imaging system may be used which further minimises the cost. In addition, paper is generally opaque at optical wavelengths which further increases the effectiveness of visible light for this purpose. However, other, invisible wavelengths may alternatively be used such as infra-red or ultraviolet wavelengths.

The means for projecting radiation may be fitted with a filter. It may be advantageous to use different coloured lighting since different wavelengths of light may pass through the hole more or less easily. For example, blue light may show a brighter hole than red light since blue light may pass more easily through the hole or else be more easily detectable by the instrument.

The means for projecting radiation on the paper may comprise means for pulsing the projected radiation (such as a radiation pulser or a strobe). Such pulsing, often referred to as strobing, may enable fast sampling. Thus the sample of paper may be merely an illuminated portion of a much larger roll. In this embodiment samples may be analysed as they pass through the permeability determination system.

The sample of paper may be disposed between the imaging device and the means for projecting radiation. This lighting arrangement is generally known as "back-lighting". By using back-lighting in this manner, any holes in the paper will be clear in an image taken by an imaging device such as a camera. Holes will appear as bright spots, or areas, in an image, or as dark spots in a negative.

Alternatively radiation may be projected on a first side of the sample of paper, and the imaging device may be operable to produce an image of the first side of the sample of paper. This lighting arrangement is generally known as "front-lighting". In contrast to back-lighting, any holes in the paper will be apparent in an image as dark spots, or areas, or as bright spots in a negative. Front-lighting may be preferable over back-lighting in some embodiments as there are no flare or halo type effects due to diffraction of light through the hole; these effects may be present in back-lighting systems. Thus, front-lighting may be able to provide a more accurate measurement of hole area.

Alternatively the means for projecting radiation may be arranged to project on a first side of the sample of paper, and a further means for projecting radiation may be arranged to project on a second side of the sample of paper. In this lighting arrangement there exists the capability for both front and back-lighting of the sample of paper. It may be advantageous to have a method which takes a low magnification image with back-lighting, in order to highlight bright spots indicating holes, and then takes a high magnification image with front lighting in order to accurately determine the size of the holes. In addition it may be advantageous to introduce a redundancy to the device, should one of the lighting systems fail.

The means for projecting radiation may comprise a mirror-switching mechanism. The mirror-switching mechanism project the radiation onto both sides of the sample of paper. Alternatively separate lighting systems may be provided for each side of the paper.

The permeability determination system may further comprise means for varying the intensity of the projected radiation (such as an intensity variation arrangement). This may be done, for example, based on the overall brightness of the image. For example, if there are large holes present in the sample it may be desirable to reduce the intensity of the radiation to avoid camera saturation.

The intensity of the projected radiation may also be varied in dependence on the paper thickness. When the device is operating in the back-light embodiment, the effectiveness of the hole measurement system may be impaired unless the intensity of light can be varied. If thicker paper is used then the visibility of holes may be decreased; this may be particularly pronounced for small, single pixel holes. Similarly, decreasing the thickness of the paper may adversely affect the contrast between holes and the background paper unless the intensity of light is adjusted to compensate. Intensity may be varied in dependence on paper thickness in accordance with a calibrated relationship, since this relationship may be non-linear. This system may be improved if provided with means for measuring the thickness of the paper, such as a gauge.

The permeability determination system may comprise a sample of paper and the sample of paper may be cigarette paper. Cigarette paper is a particularly appropriate type of paper for this manner of permeability determination since its permeability must be closely controlled in order to ensure the correct amount of chemicals are transferred to the user during smoking.

The system may comprise data storage means which may be arranged to store data relating to the correlation between number and/or area of holes, and permeability. The permeability determination system may be arranged to read from the data storage device in order to determine the contribution of the detected holes to the permeability of the sample. The permeability determination system may also be arranged to write to the data storage device, as more data relating to this correlation are discovered.

Providing data storage means also allows the device to store data relating to the holes measured, and/or their contribution to the permeability of the sample. Building statistics may be advantageous for analysing the average permeability of samples of paper and the standard deviation of permeability. Specifically an analysis of the relative positions and/or sizes of holes measured in samples may point towards a specific fault or quality issue.

The data storage means are arranged to store the permeability of paper, where the permeability stored may be that of (substantially) hole-free paper. Storing the permeability of hole-free paper further allows permeability of the sample of paper to be calculated by combining the inherent permeability of paper with the contribution made by holes.

Preferably the imaging device is operable to produce a digital image. By using digital imaging, any resulting images may be analysed on a pixel-by-pixel basis. This has the advantage of facilitating the use of electronic analysis routines for fast analyses. Typically the imaging device will be a camera but a line-scan camera, scanner or other imaging device could alternatively be used.

The permeability determination system may further comprise means for relating the brightness of at least one pixel to hole size. Many holes in a sample of paper may occupy more than one pixel in the image produced by the imaging device. Generally it will be straightforward to determine the area of these holes by knowing the resolution of the image and the size of the sample in the field of view. However, for holes occupying only a single pixel in the image it will be more difficult to accurately determine hole size. By relating pixel brightness to hole size it may be possible to determine the actual size of a hole.

This technique can also be used for holes occupying more than one pixel since it may be difficult to determine precisely the edges of the holes. Using this technique it may be possible to relate pixel brightness to the degree of obscuration, and therefore to the hole size.

The means for relating the brightness of at least one pixel to hole size may utilise calibration data. The relationship between pixel brightness and hole size may be calibrated and stored in the permeability determination system, for example in the data storage means. This can allow accurate determination of the size of a hole.

The permeability determination system may further comprise means for indicating whether the contribution of detected holes to the permeability of the sample of paper meets predefined criteria. This feature can allow the permeability determination system to provide a pass/fail indication for the sample of paper under examination. The storage device may be arranged to store data relating to acceptable upper and/or lower thresholds of acceptable permeability. If the sample falls outside of predefined acceptable thresholds then there may be an indication that the sample is unacceptable and should be rejected. This could be signalled to an operator by a visual or audible indicator.

Preferably a processor is provided. Use of a processor enables digital processing and fast analysis of data using conventional techniques. The processor may be provided by a computer in communication with the imaging device. Alternatively, the processor may be provided integrally with the imaging device.

By using the imaging system together with conventional means for directly measuring permeability, the relationship between holes and permeability may be determined. Thus the pressure differential system may be used as a part of a calibration system where the correlation between holes and permeability may be ultimately determined. In such a calibration system, the permeability of hole-free paper may be measured and then the permeability of paper comprising holes may be measured. Through a series of experiments the correlation between number and area of holes, and permeability may be determined. Once determined, the correlation data may be stored in more basic permeability determination systems that do not comprise means for creating pressure differentials, so that those devices can make correlations based on experimental data.

Therefore, according to another aspect of the present invention there is provided a calibration system for determining a relationship between holes in a sample of paper and the permeability of the paper, the calibration system comprising: means for creating a pressure differential across the sample of paper means for measuring airflow; and a permeability determination system as previously defined.

The sample of paper may be selected based on the sizes and/or number of holes. By specifically selecting the sample of paper based on the holes there present, a correlation between holes and permeability may be rapidly built up. This may be of particular relevance in the calibration system. The sample of paper may also be selected from areas likely to be of the highest and lowest permeabilities; thus establishing greater certainty that the sample falls within any required limits.

According to another aspect of the present invention there is provided a method of determining the permeability of a sample of paper comprising the steps specified in claim 15.

Any of the apparatus features may be provided as method features and *vice versa*.

**Brief Description of the Drawings**

Preferred features of the present invention will now be described, purely by way of example, with references to the accompanying drawings, in which:

Figure 1 shows the construction of a typical cigarette;

Figure 2 shows an image of typical cigarette tipping paper;

Figure 3 shows a permeability determination system in an embodiment of the invention;

Figure 4 is a diagrammatic representation of a histogram of brightness for an image of a faulty paper sample;

Figure 5 shows standard pressure differential apparatus including an optical hole determination system in an embodiment of the invention;

Figure 6 shows a flow diagram for use in an embodiment of the invention; and Figure 7 shows a permeability determination system for use in a fast sampling embodiment of the invention.

**Permeability of Cigarette Papers**

There are many different types of paper used in the manufacture of a single cigarette. Each paper has a particular function within the overall design; in particular these papers are used to control the burning characteristics of the cigarette and the dilution of smoke delivered to the user. The functioning of the cigarette is thus reliant on carefully selected paper permeability. It is desirable to measure these permeabilities accurately and quickly to ensure that the quality of the products is controlled.

Permeability is a measurement of the ability of a fluid to pass through a porous material. This parameter is usually expressed in CORESTA units (CU): this relates to the volume of air in cubic centimetres that passes through a square centimetre of the material in one minute at a pressure of 1kPa.

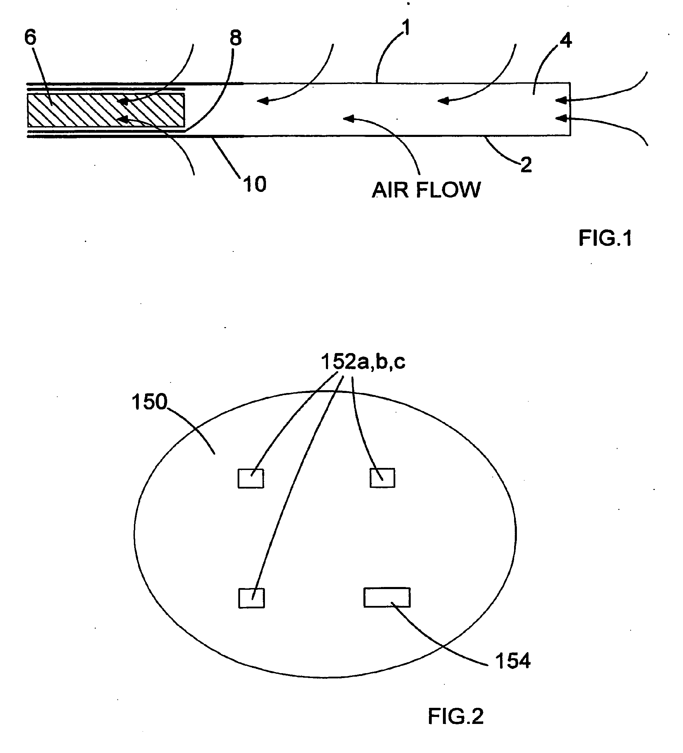

Figure 1 shows the anatomy of a cigarette 1.

The cigarette comprises a filter 6 and tobacco 4, held together by a number of different types of paper. Envelope paper 2 cylindrically surrounds the tobacco and is designed to control the burning characteristics of the lit coal. The filter 6 is completely cylindrically surrounded by plug wrap paper 8: a material with a particularly high permeability of around 40,000 CU. Filter 6 and plug wrap paper 8 are cylindrically surrounded by tipping paper 10, and the tipping paper also extends beyond the filter to surround a portion of the tobacco 4. Tipping paper is normally of low permeability but nonetheless is often deliberately perforated, either mechanically or by means of a laser. These created holes are designed to increase the permeability of the paper in specific areas so that air mixes with the smoke ahead of the filter thereby controlling the cigarette "taste" and the delivery of tar and nicotine to the user. An example of deliberately perforated tipping paper 150 is shown in Figure 2. The paper comprises holes 152 a,b,c and 154 cut in it by a laser. Hole 154 is accidentally mis-shaped; this may change smoke dilution characteristics. It is particularly important that the permeability of these papers is known so that the delivery of tar and nicotine is consistent and predictable.

Permeability of paper is normally measured by clamping the paper under test in a clamp and then creating a pressure differential across the paper. Typically the clamped paper will form a boundary between two chambers of different pressure potential. The pressure differential between the two chambers is set to a required level and the air flow is measured. The flow rate per unit area at a particular pressure differential is then calculated yielding the paper permeability. Alternatively the pressure differential can be changed until a certain air flow rate is reached.

This method of measuring paper permeability is slow and expensive. The combination of these two factors renders it impossible from a practical point of view to measure the permeability of all paper that is used in the manufacture of a cigarette. Therefore one is restricted to measuring random samples as part of a quality control process. This means that the presence of random unwanted holes of indeterminate size may go undetected leading to inferior final products.

**Embodiments of the Invention**

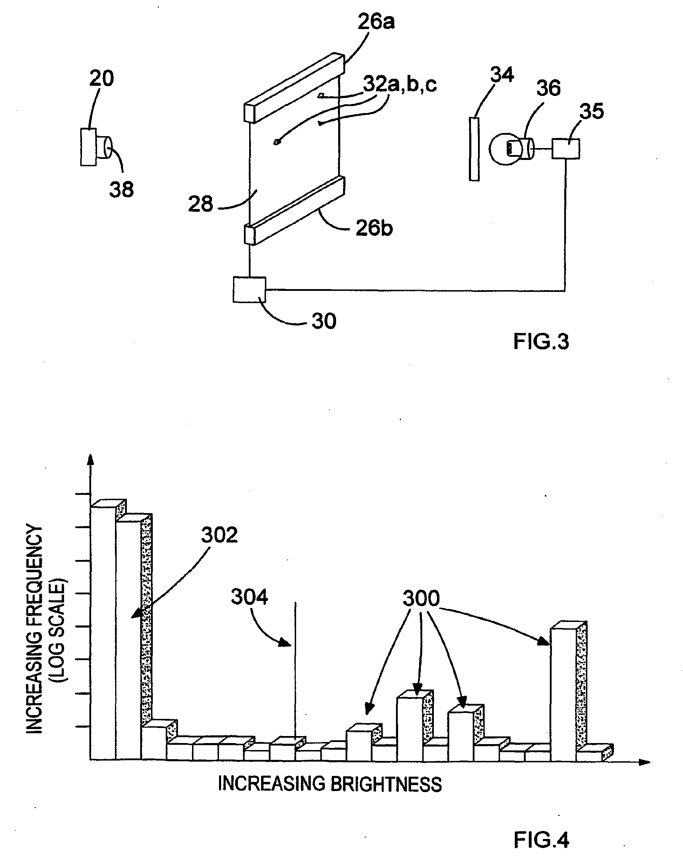

Figure 3 shows a permeability determination system for determining the permeability of a sample of paper. In a first embodiment of the invention a camera 20 is arranged to image holes 32a,b,c which may be present in a sample of paper 28. This is done in order to determine the permeability of the sample; wherein the permeability of the sample is contributed to by the inherent permeability of the paper and any holes present. (The inherent permeability of paper is determined by the materials used, the finish, thickness and weave of the paper, among other factors.)

A sample of cigarette paper 28 (which may be any type of cigarette paper), comprising holes 32a,b,c is held by clamps 26a and 26b along opposite edges of the sample. The holes present in the paper may be present as a result of a manufacturing flaw, or else may be deliberately cut into the paper mechanically or by a laser. (It is more common to cut deliberate holes in tipping paper than in other cigarette papers). Whether the holes in the paper are there by accident or otherwise it is desirable to measure their presence and to determine the effect that they have on the permeability of the paper. Even if holes are present deliberately there may be errors in the position or shape of the holes which can change the permeability of the sample.

A digital camera 20 is arranged to point at one of the faces of the sample of paper and a light 36 is arranged to point at the other face of the paper. Since the light 36 is on the opposite side of the paper to the camera, it is known as a back-light.

In operation of the permeability determination system, firstly back-light 36 is arranged to turn on and secondly camera 20 takes an image of the entire sample of paper. The image taken by camera 20 is then analysed by control electronics within the camera, and pixel brightness is measured for each pixel within the field of view.

In this way a histogram of brightness can be created for the field of view giving the total number of pixels contained in each defined brightness bin. Pixels that correspond to holes contribute to maxima (in a back-lighting embodiment) in the histogram of brightness. In this way the pixels contributing to a maximum may be identified together with their associated coordinates.

Once holes have been identified, a single hole is brought to the centre of the field of view either manually or automatically. The lens 38 is changed for a high magnification lens and an image of the hole is obtained that fills a significant proportion of the field of view. At this point the image can be processed and an area calculated for the perforation. This is repeated for all holes detected in the field of view.

Many of the holes detected in the sample of paper may occupy more than one pixel in the image produced by the imaging device. Generally it will be straightforward to determine the area of these holes by knowing the resolution of the image and the size of the sample in the field of view. However, for holes occupying only a single pixel, even in a magnified image, it will be difficult to determine hole size accurately.

A pixel in a Charge Coupled Device (CCD) corresponds to a physical area of paper; this area can be calculated in dependence on the resolution of the camera's CCD and the size of the sample in the camera's field of view. For a single pixel hole, if the pixel brightness were the same as the pixel brightness in a flat field then one might expect that the hole area would just be the area of paper that corresponds to the pixel size. Correspondingly one might expect that if a pixel were only half the brightness of the pixel brightness in a flat field, then the hole would only have half of the area that corresponds to a pixel. In fact, the relationship is not quite linear and a calibration curve is required to relate pixel brightness to the actual size of the hole. In this way, by relating pixel brightness to hole size it is possible to determine the actual size of every hole. (By way of background, a flat field is an image taken with the back-light on but no paper sample present).

This technique can also be used for holes occupying more than one pixel since it may be difficult to determine the edges of the holes precisely. Pixel brightness may be related to the degree of obscuration, and therefore to the hole size.

By use of the described technique it is possible to determine the number of holes, and the area of each of the holes in the sample of paper. Camera 20 has an internal memory comprising calibration data relating the size and number of holes to paper permeability. These data may be derived from experiment, or else may be derived theoretically: if the intrinsic permeability of the paper is known then it may be possible to calculate the effect of an absence of an area of paper i.e. a hole. In this way, the contribution of any holes present in the sample of paper to the permeability of the sample of paper may be calculated. For these reasons it may be beneficial to store the intrinsic permeability of the paper in memory.

The calculated permeability may then be compared to upper and lower permeability thresholds stored in memory and the paper sample may be rejected as a result. Alternatively a figure of merit (FoM) may be calculated as a percentage where:

$$FoM = \frac{D * N_{pix}}{N_{BG}}$$

. D is the difference in brightness level from background; $N_{pix}$ is number of pixels with brightness greater than background; and $N_{BG}$ is number of pixels with background brightness. If the FoM is above an acceptable threshold then the sample of paper may be rejected; it may be that 3E-6 or some similar number is an acceptable threshold value for these purposes. Where a sample of paper does not fall within stored threshold limits then this may signalled to an operator by an audible or visual indicator.

This system may be used to measure what permeability would be at standard temperature and pressure irrelevant of the actual conditions. Therefore, it may be considered to be independent of temperature, pressure and humidity.

Although any holes 32a,b,c may be visible in the absence of back-light 36, the light increases the contrast between the holes and the paper, and makes holes easier to identify. In order to maintain the contrast at a desirable level clamp 26b is connected to a measurement gauge 30. Measurement gauge 30 measures the distance between the opposing jaws of clamp 26b. Thus, when paper is held in the clamp, the measurement gauge measures the width of the paper. Measurement gauge 30 provides an output of the width of the paper which is received by a controller 35 of the back-light 36. Controller 35 adjusts the power of the back-light 36 in dependence on the width of the paper in the clamp. This adjustment of back-light power is dependent on paper thickness and may be linear or non-linear.

If the paper held in the clamps 26a and 26b is thin then some of the light the back-light pass through the paper and cause it to "glow", as viewed from the other side. Of course paper will normally "glow" if lit from the back. However, this is only tolerated up to a certain limit since if the "glow" is too bright then it will reduce the contrast between the holes and the paper, and will make holes more difficult to detect. Conversely, if the paper is thick then it is desirable to increase the power of back-light since small holes in particular are likely to become obscured and go undetected if the illumination is too weak.

Back-light 36 may be fitted with a removable filter 34 in order to change the colour of the output light. Moreover back-light 36 need not be at optical wavelengths and in some embodiments it may be desirable to use an infra-red or ultraviolet back-light.

Although the foregoing has been described with reference to detection of actual holes, this is merely illustrative of an embodiment of the invention. Rather the permeability of a sample of paper 28 may be determined only by the brightness of the image produced by camera 20.

As mentioned, a histogram of brightness can be created for the field of view of camera 20 giving the total number of pixels contained in each defined brightness bin. An example of such a histogram is shown pictorially in figure 4. The brightness of the background paper is generally consistent across the sample such that all pixels are located in the first two bins 302. Areas of brightness may correspond to perforations in the paper and these will be identifiable as peaks in the histogram 300. (This is with reference to figure 2 where the paper is lit with a back-light. If a front light or ambient lighting were used then perforations would be identifiable as minima as opposed to maxima in the histogram). Any individual hole may cause several pixels of different brightness in the focal plane of camera 20. In this way a hole may have an effect on the populations of several bins in the histogram 300.

Pixel brighmess may be related to the area of hole imaged by that pixel. In turn, this may be related to the effect of that hole on the permeability of the sample of paper. This may be done either by calibration, or else theoretically: if the inherent permeability of paper is known then the effect of the absence of an area of paper is calculable.

Using this method the number of pixels in a particular brightness bin may be found, and their effect on the permeability of the sample of paper can be calculated without having to determine directly the area of any hole. In practice a peak pixel brightness corresponds to those pixels that only image an area of hole in the sample (and not any obscuring paper). Pixels below this peak brightness but above a threshold 304 correspond to pixels that image an area of hole and also an area of obscuring paper. Pixels below the threshold are unlikely to correspond to holes in the sample of paper 28. However, they may correspond to areas of low paper thickness. Therefore, the threshold may be lowered to further enable detection of inhomogeneities in the paper thickness and the contribution of those to the permeability of the sample.

Further still, the use of histograms is merely illustrative of another embodiment of the invention. As an alternative it is possible to determine the mean pixel illumination across the field of view of the camera 20. This will be especially appropriate where the power of back-light 36 is varied in dependence on the thickness of paper 28, as measured by measurement gauge 30. Back-light power may be sensitively tuned such that the background brightness of paper 28, as imaged by camera 20, is constant independent of paper thickness. Thus, any holes present in the paper 28 will have a brightness above the background level which will affect the mean pixel brightness across the field of view. In this way the deviation in mean pixel brightness from the background brightness of the paper may be related to the area of holes in the sample. In turn this may be related to the contribution of those holes to the permeability of the sample of paper.

This system may be preferable in some embodiments since it allows use of a low resolution camera, fast measurement and easy data analysis.

## Calibration System

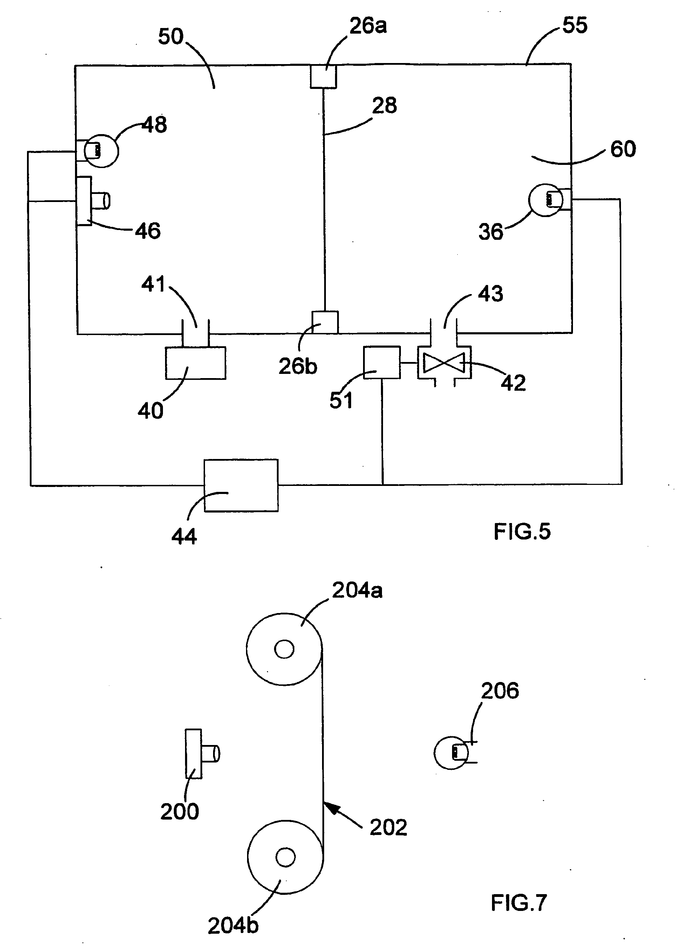

Figure 5 shows part of a calibration system where the relationship between holes and paper permeability is determined. As part of the calibration system a pressure differential unit 55 is used to measure the permeability of a sample of paper 28.

The pressure differential unit 55 comprises two chambers 50,60 separated by a sample of paper 28 which is clamped in place along two sides by clamps 26a and 26b. Each chamber has a valve 41,43 and chamber 50 also has a pump 40 comprising a pressure meter. Pump 40 is arranged to create a pressure $P_{50}$ in chamber 50. Chamber 60 is open to the atmosphere via valve 43 and therefore has a pressure of close to 1 atmosphere. The pressure $P_{50}$ is not equal to 1 atmosphere and thus a pressure differential is created across paper 28: $P_{50}$ may be less than 1 atmosphere in which case the pump "sucks" through the paper; or else $P_{50}$ may be greater than 1 atmosphere in which case the pump "blows" through the paper. In either case, the natural permeability of the paper causes a flow of air from the higher pressure chamber to the lower pressure chamber. Clearly this flow of air will be greater for high permeability paper, and will be greater still if the paper contains any holes.

Chamber 60 has a valve 43 which is open to the atmosphere. Fan 42 is situated within valve 43 and does not create any substantial resistance to the flow of air through valve 43. Fan 42 is connected to speed sensor 51 which measures the flow rate through the fan. Knowing the cross-sectional area of the valve and the paper permits the measured flow rate through the valve to be easily related to the flow rate per unit area through the paper - i.e. the permeability. To this end speed sensor 51 is electrically connected to computer 44 for analysis of the data and determination of the permeability of the sample of paper.

It is worth mentioning that this is only one way in which permeability may be measured. Rather than setting $P_{50}$ and measuring the resulting flow rate it would be equally possible to vary $P_{50}$ until a desired air flow rate per unit area is reached; this would yield the paper permeability equally effectively.

It is also worth mentioning that there are many other ways that a pressure differential unit could be arranged. For example, each chamber could comprise a pump. An analysis of the work done by each pump to maintain the pressure differential could then be related to a flow rate through the paper, yielding the paper permeability. A possible advantage of such a technique is that it may make measurements more precise (since atmospheric pressure varies on a daily basis). In addition it may enable the control of other parameters such as temperature and humidity.

Chamber 50 also comprises a digital camera 46 mounted on a wall facing the clamped paper 28. Camera 46 comprises a "zoom" functionality and is arranged in electrical connection with computer 44. Computer 44 controls the operation of, and analyses data from, the camera 46. Computer 44 is also arranged to control the operation of front-light 48 and back-light 36. Front-light 48 is located adjacent to camera 46 in chamber 50 directly opposite clamped paper 28. In operation front-light 48 is designed to give an even illumination of the entire surface of the paper. In another embodiment it may be desirable to have more than one light in order to achieve a more even illumination. Back-light 36 is mounted on a wall of chamber 60 facing clamped paper 28; back-light 36 is equally designed to give an even illumination of the paper in normal operation. Both lights 48,36 may be fitted with filters to change the colour of their output if desired.

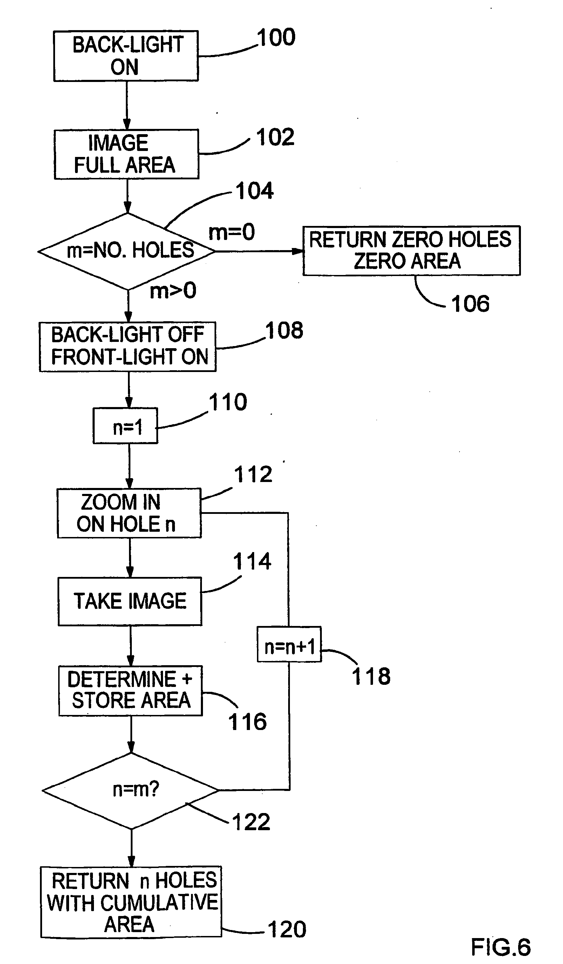

In normal operation of this system, the permeability of the paper is measured with the pressure differential equipment, and the results are stored in the memory of computer 44. Either before, during or after this measurement the sample of paper is examined for holes by the optical apparatus. The flow diagram for this process is shown in Figure 6, as controlled by the computer 44 in software or firmware.

At step 100 back-light 36 is turned on in order to illuminate the sample of paper. An image is then taken of the entire paper sample, at step 102, by camera 46. The digital image produced by the camera 46 is then sent for analysis to the computer 44. This digital image is then examined at step 104 for bright areas which may correspond to perforations in the paper. If there are no bright areas then the routine returns a result of "no holes" and ends; otherwise it returns a result "m" equal to the number of holes detected together with the co-ordinates of their centres. Back-light 36 is then turned off and front-light 48 is turned on at step 108, for higher accuracy analysis of the shape and area of holes.

Front-lighting may be more suitable for higher accuracy analysis since back-lighting may produce unwanted effects due to the diffraction of light through holes. Such effects, commonly known as "flare" or "halo" effects are eliminated in front lighting systems. However, backlighting generally produces a greater contrast between holes and the background and is therefore useful for finding the locations of holes.

Step 110 introduces parameter "n" (and equates it to 1) which is used in a loop for examining each of the detected holes in turn. The zoom functionality of camera 46 is then utilised at step 112 and the camera 46 focuses on the centre of the first detected hole, and takes an image at step 114. Step 116 then determines the area of the first hole according to techniques already described and stores this area in computer 44; of course, in the front-lighting arrangement holes are detectable as dark areas against a bright background. Step 122 carries out a simple assessment of whether n=m: if not then n is augmented by one and the loop returns to step 112; if so then the flow diagram returns the result that there are "m" holes, with the area of each hole, and the cumulative area of holes in the sample of paper.

This may be referred to as a calibration system. The permeability of the sample of paper is measured with differential pressure means and then the number and area of holes is measured by optical means. Through a series of experiments the correlation between number of holes, area of holes, and permeability may be determined. This series of experiments would naturally involve analysing the permeability of hole-free paper and also paper with many different shapes and numbers of holes. Upon determination of correlation data, these data may be stored in other systems that do not comprise means for creating pressure differentials such as the system shown in figure 3. This provides a method for determining the permeability of samples of paper based only on the number and area of holes present.

It may be that the calibration system needs to be individually performed for each different type of paper, but alternatively a relationship may be found between the permeabilities of different types of paper rendering this needless. It is likely that any experiments would occur at standard temperatures and humidities so that any potential differences in measurement produced by variations in these parameters can be eliminated.

Although the calibration system is described above with an imaging system for determining the number and area of holes, alternatives exist. For example, the hole formation in the sample of paper may be known, and need not be determined by optical means. For hole formation to be reliably known, there must a high reliability in the size and number of holes present in the sample. To this end holes are cut in the sample by high integrity means, which may be a reliable laser or a reliable mechanical cutting system.

### Second Embodiment

In a second embodiment of the present invention a fast sampling system is designed to determine the permeability of paper.

Referring to Figure 7, Paper 202 (which is cigarette tipping paper) is wound on reel 204a and is transferred onto reel 204b by clockwise rotation of the two reels. A train of paper bridges the separation between the two reels and paper may be analysed for holes as it travels in this train.

Camera 200 points at one side of paper 202 and back-light 206 points at the reverse side of the paper 202. It is desirable that the entire length of paper is examined for holes so that the consistency of the permeability of the paper may be determined. To this end, back-light 206 is a strobe light. Camera 200 may either be electrically linked to the strobe light to ensure that it takes pictures at the same frequency as the strobe pulses, or else the camera may have a light activated shutter. Either way, every time the strobe pulses the camera takes an image and analyses any holes with an internal processor. Any holes detected in the paper are stored in the camera's internal memory, and their contribution to the permeability of the paper is calculated according to the techniques described above.

It may be desirable to select the frequency of the strobe and the speed of the paper train so that all of the paper is imaged by the camera. To do this, it may be prudent to provide some overlap between the sections of paper imaged in sequent exposures. Alternatively it may be more desirable to have a number of camera-strobe pairs (or even just a plurality of cameras but with a single strobe) operating in parallel in order to produce full sampling of the paper while keeping the speed of the paper train high.

It will be appreciated that the description of a paper train is purely illustrative and that the aforementioned technique could equally be applied to a succession of discrete samples.

Although the present techniques are particularly applicable to cigarette papers, they are not so uniquely confined. The permeable property of paper is used in other filtering applications for both gases and liquids and a method of detecting holes and measuring permeability in these papers is also desirable.

### Claims

1. A permeability determination system comprising:

    an imaging device (20,200), operable to produce an image of a sample of paper (28), means for determining the brightness of the image data analysis means for detecting at least one area in the images which corresponds to a hole (32a,b,c) in the sample of paper (28), said at least one area contrasting with the normal background brightness of the sample of paper;

**characterised in that** it further comprises

    data storage means arranged to store the inherent permeability of the substantially hole-free paper; and
    data analysis means for determining the permeability of the sample of paper based on the inherent permeability of the paper as stored in the data storage means, and the contribution to permeability of any detected holes.

2. A permeability determination system according to claim 1 further comprising data analysis means for determining the area of detected holes.

3. A permeability determination system according to claim 1 or claim 2 further comprising data analysis means for determining the shape of detected holes.

4. A permeability determination system according to any of the preceding claims wherein the magnification factor for images produced by the imaging device (20,200) is variable.

5. A permeability determination system according to any of the preceding claims further comprising means for projecting radiation (36,206) on the sample of paper (28).

6. A permeability determination system according to claim 5 wherein the sample of paper (28) is disposed between the imaging device (20,200) and the means for projecting radiation (36,206).

7. A permeability determination system according to claim 5 further comprising means for projecting radiation (48) on a first side of the sample of paper, wherein the imaging device (46) is operable to produce an image of the first side of the sample of paper.

8. A permeability determination system according to claim 5 wherein means for projecting radiation (36,48) are arranged to project radiation on a first side and a second side of the sample of paper (28).

9. A permeability determination system according to claim 8 wherein the imaging device (46) is arranged to take a lower resolution image of the sample of paper (28) when the means for projecting radiation (36) is arranged to project radiation on the first side of the sample of paper than when the means for projecting radiation is arranged to project radiation (48) on the second side of the sample of paper.

10. A permeability determination system according to any of claims 6 to 9 wherein the intensity of the projected radiation is variable.

11. A permeability determination system according to claim 10 wherein the intensity of the projected radiation is variable in dependence on the paper thickness.

12. A permeability determination system according to any of the preceding claims wherein the imaging device (46) is operable to produce a digital image.

13. A permeability determination system according to claim 12 further comprising means for relating the brightness of at least one pixel to hole size (44).

14. A calibration system for determining a relationship between holes in a sample of paper and the permeability of the paper, the calibration system comprising:

    means for creating a pressure differential (55) across the sample of paper (28); means for measuring airflow (51), and a permeability determination system according to any of the preceding claims.

15. A method of determining the permeability of a sample of paper comprising the steps of:

    producing an image of a sample of paper (28); determining the brightness of the image; detecting at least one area in the image which corresponds to a hole (32a,b,c) in the sample paper (28), said at least one area contrasting with the normal background brightness of the sample of the paper **characterised in that** it further comprises the steps of retrieving a measure of the inherent permeability of the substantially hole-free paper from data storage means; and determining the permeability of the sample of paper based on the inherent permeability of the paper as retrieved from the data storage means and the contribution to permeability of any detected holes.

**Patentansprüche**

1. System zur Durchlässigkeitsbestimmung, umfassend:

    eine bildgebende Vorrichtung (20, 200), geeignet zur Erzeugung von Bildern einer Papierprobe (28),
    Mittel zur Bestimmung der Helligkeit des Bildes, Datenanalysemittel zur Detektion zumindest eines Bereiches im Bild, der einem Loch (32a, b, c) in der Papierprobe (28) entspricht, wobei zumindest dieser eine Bereich einen Kontrast zur normalen Hintergrundhelligkeit der Papierprobe (28) aufweist,

    **dadurch gekennzeichnet, dass** es darüber hinaus unfasst
    Datenspeichermittel zur Sicherung der inhärenten Durchlässigkeit des im Wesentlichen lochfreien Papiers und
    Datenanalysemittel zur Bestimmung der Durchlässigkeit der Papierprobe, basierend auf der in den Datenspeichermitteln abgelegten inhärenten Durchlässigkeit des Papiers und dem Einfluss etwaig erkannter Löcher auf die Durchlässigkeit.

2. System zur Durchlässigkeitsbestimmung nach Anspruch 1 weiters umfassend Datenanalysemittel zur Bestimmung der Fläche erkannter Löcher.

3. System zur Durchlässigkeitsbestimmung nach Anspruch 1 oder 2 weiters umfassend Datenanalysemittel zur Bestimmung der Kontur erkannter Löcher.

4. System zur Durchlässigkeitsbestimmung nach einem der vorangehenden Ansprüche wobei der Vergrößerungsfaktor für Bilder der bildgebenden Vorrichtung (20, 200) variabel ist.

5. System zur Durchlässigkeitsbestimmung nach einem der vorangehenden Ansprüche weiters umfassend Mittel zur Bestrahlung der Papierprobe.

6. System zur Durchlässigkeitsbestimmung nach Anspruch 5, wobei die Papierprobe (28) zwischen der bildgebenden Vorrichtung (20, 200) und den Mitteln (36, 206) zum Bestrahlen angeordnet ist.

7. System zur Durchlässigkeitsbestimmung nach Anspruch 5, weiters umfassend Mittel (48) zur Bestrahlung einer ersten Seite der Papierprobe, wobei die bildgebende Vorrichtung (46) geeignet ist, ein Bild von der ersten Seite der Papierprobe zu erstellen.

8. System zur Durchlässigkeitsbestimmung nach Anspruch 5, wobei die Mittel (36 ,48) zur Bestrahlung zum Bestrahlen einer ersten und einer zweiten Seite der Papierprobe angeordnet sind.

9. System zur Durchlässigkeitsbestimmung nach Anspruch 8, wobei die bildgebende Vorrichtung (46) angeordnet ist, um ein Bild der Papierprobe mit geringerer Auflösung zu erstellen, wenn die Mittel (36) zur Bestrahlung die erste Seite der Papierprobe bestrahlen, als wenn die Mittel (48) zur Bestrahlung die zweite Seite der Papierprobe bestrahlen.

10. System zur Durchlässigkeitsbestimmung nach einem der Ansprüche 6 bis 9, wobei die Intensität der Bestrahlung variabel ist.

11. System zur Durchlässigkeitsbestimmung nach Anspruch 10, wobei die Intensität der Bestrahlung in Abhängigkeit der Papierdicke variabel ist.

12. System zur Durchlässigkeitsbestimmung nach einem der vorangehenden Ansprüche, wobei die bildgebende Vorrichtung (46) zur Erzeugung eines digitalen Bildes geeignet ist.

13. System zur Durchlässigkeitsbestimmung nach Anspruch 12, weiters Mittel (44) zum Beziehen des Helligkeitswertes zumindest eines Bildpunktes auf das Lochmaß.

14. Kalibriersystem zur Bestimmung der Relation zwischen Löchern in einer Papierprobe und der Durchlässigkeit des Papiers, wobei das Kalibriersystem umfasst:

Mittel zur Erzeugung eines Druckunterschiedes (55) über die Papierprobe (28),
Mittel zur Messung der Luftströmung (51) und ein System zur Durchlässigkeitsbestimmung nach einem der vorangehenden Ansprüche.

15. Verfahren zur Bestimmung der Durchlässigkeit einer Papierprobe, umfassend die Schritte;

Erzeugung eines Bildes der Papierprobe (28),
Bestimmung der Bildhelligkeit,
Detektion zumindest eines Bereiches im Bild, der einem Loch (32a, b, c) in der Papierprobe (28) entspricht, wobei zumindest dieser eine Bereich einen Kontrast zur normalen Hintergrundhelligkeit der Papierprobe (28) aufweist,

**dadurch gekennzeichnet, dass** es darüber hinaus die Schritte umfasst:

Abfragen einer Messung der inhärenten Durchlässigkeit des im Wesentlichen lochfreien Papiers aus gespeicherten Daten und
Bestimmung der Durchlässigkeit der Papierprobe, basierend auf der inhärenten Durchlässigkeit des Papiers, wie sie aus dem Datenspeicher abgefragt ist und auf dem Einfluss auf die Durchlässigkeit beliebiger erkannter Löcher.

**Revendications**

1. Système de détermination de la perméabilité comprenant:

un dispositif d'imagerie (20, 200) apte à produire une image d'un échantillon de papier (28),
un moyen pour déterminer la luminosité de l'image,
un moyen d'analyse de données pour détecter au moins une zone dans l'image qui correspond à un trou (32a, b, c) dans l'échantillon de papier (28), ladite au moins une zone contrastant avec la luminosité normale de l'arrière-plan de l'échantillon de papier;

**caractérisé en ce qu'**il comprend en outre
des moyens de stockage de données agencés pour stocker la perméabilité inhérente du papier sensiblement exempt de trous;
un moyen d'analyse de données pour déterminer la perméabilité de l'échantillon de papier basée sur la perméabilité inhérente du papier telle que stockée dans le moyen de stockage de données, et la contribution à la perméabilité de tout trou détecté.

2. Système de détermination de perméabilité selon la revendication 1, comprenant en outre un moyen d'analyse de données pour déterminer la zone des trous détectés.

3. Système de détermination de perméabilité selon la revendication 1 ou la revendication 2, comprenant en outre un moyen d'analyse de données pour déterminer la forme des trous détectés.

4. Système de détermination de la perméabilité selon l'une quelconque des revendications précédentes, dans lequel le facteur d'agrandissement des images produites par le dispositif d'imagerie (20, 200) est variable.

5. Système de détermination de la perméabilité selon l'une quelconque des revendications précédentes, comprenant en outre un moyen pour projeter un rayonnement (36, 206) sur l'échantillon de papier (28).

6. Système de détermination de la perméabilité selon la revendication 5, dans lequel l'échantillon de papier (28) est disposé entre le dispositif d'imagerie (20, 200) et le moyen pour projeter le rayonnement (36, 206).

7. Système de détermination de la perméabilité selon la revendication 5, comprenant en outre un moyen pour projeter le rayonnement (48) sur un premier côté de l'échantillon de papier, où le dispositif d'imagerie (46) est apte à produire une image du premier côté de l'échantillon de papier.

8. Système de détermination de la perméabilité selon la revendication 5, dans lequel des moyens de projection de rayonnement (36, 48) sont agencés pour projeter un rayonnement sur un premier côté et un second côté de l'échantillon de papier (28).

9. Système de détermination de la perméabilité selon la revendication 8, dans lequel le dispositif d'imagerie (46) est agencé pour prendre une image d'une résolution plus basse de l'échantillon de papier (28) lorsque le moyen de projection de rayonnement (36) est agencé pour projeter le rayonnement sur le premier côté de l'échantillon de papier que lorsque le moyen de projection de rayonnement est agencé pour projeter le rayonnement (48) sur le second côté de l'échantillon de papier.

10. Système de détermination de la perméabilité selon l'une quelconque des revendications 6 à 9, dans lequel l'intensité du rayonnement projeté est variable.

11. Système de détermination de la perméabilité selon la revendication 10, dans lequel l'intensité du rayonnement projeté est variable en fonction de l'épaisseur du papier.

12. Système de détermination de la perméabilité selon l'une quelconque des revendications précédentes, dans lequel le dispositif d'imagerie (46) est apte à produire une image numérique.

13. Système de détermination de la perméabilité selon la revendication 12, comprenant en outre un moyen pour mettre en rapport la luminosité d'au moins un pixel avec la taille du trou (44).

14. Système de calibrage pour déterminer une relation entre des trous dans un échantillon de papier et la perméabilité du papier, le système de calibrage comprenant:

un moyen pour créer un différentiel de pression (55) dans l'échantillon de papier (28);
un moyen pour mesurer un écoulement d'air (51); et
un système de détermination de la perméabilité selon l'une quelconque des revendications précédentes.

15. Procédé de détermination de la perméabilité d'un échantillon de papier comprenant les étapes de:

produire une image d'un échantillon de papier (28);
déterminer la luminosité de l'image;

détecter au moins une zone dans l'image qui correspond à un trou (32a, b, c) dans l'échantillon de papier (28); ladite au moins une zone contrastant avec la luminosité normale de l'arrière-plan de l'échantillon de papier;

**caractérisé en ce qu'**il comprend en outre les étapes de:

extraire une mesure de la perméabilité inhérente du papier sensiblement exempt de trous du moyen de stockage de données; et
déterminer la perméabilité de l'échantillon de papier sur la base de la perméabilité inhérente du papier telle qu'extraite du moyen de stockage de données et la contribution à la perméabilité de tout trou détecté.

FIG.1

FIG.2

FIG.3

INCREASING FREQUENCY (LOG SCALE)

INCREASING BRIGHTNESS

FIG.4

FIG.5

FIG.7

BACK-LIGHT
ON — 100

IMAGE
FULL AREA — 102

m=NO. HOLES — 104

m=0 → RETURN ZERO HOLES
ZERO AREA — 106

m>0

BACK-LIGHT OFF
FRONT-LIGHT ON — 108

n=1 — 110

ZOOM IN
ON HOLE n — 112

TAKE IMAGE — 114

n=n+1 — 118

DETERMINE +
STORE AREA — 116

n=m? — 122

RETURN n HOLES
WITH CUMULATIVE
AREA — 120

FIG.6

**EP 2 021 765 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5341824 A